# EUROPÄISCHE PATENTSCHRIFT

(11) **EP 1 257 274 B1**
(45) Veröffentlichungstag und Bekanntmachung des Hinweises auf die Patenterteilung: **12.05.2004**
(21) Anmeldenummer: 01915023.4
(22) Anmeldetag: 15.02.2001
(51) Int. Cl.: A61K 31/426, A61P 31/18

(54) **VERWENDUNG VON 2-METHYL-THIAZOLIDIN 2,4-DICARBONSÄURE ZUR HERSTELLUNG EINES MEDIKAMENTS ZUR BEHANDLUNG VON INFEKTIONSKRANKHEITEN**
USE OF 2-METHYL-THIAZOLIDIN-2,4-DICARBOXYLIC ACID FOR TREATING INFECTIOUS DISEASES
UTILISATION D'ACIDE 2-METHYL-THIAZOLIDIN-2,4-DICARBOXYLIQUE POUR LE TRAITEMENT DE MALADIES INFECTIEUSES

(30) Priorität: 15.02.2000 DE 10008050
(43) Veröffentlichungstag der Anmeldung: 20.11.2002
(73) Patentinhaber: Susilo, Rudy, Dr., 50999 Köln (DE)
(72) Erfinder: SUSILO, Rudy, 50999 Köln (DE); ROMMELSPACHER, Hans, 14193 Berlin (DE)
(74) Vertreter: Bucher, Ralf, Dipl.-Ing. (Univ.)
(86) Internationale Anmeldenummer: PCT/DE2001/000655
(87) Internationale Veröffentlichungsnummer: WO 2001/060342

(56) Entgegenhaltungen:
- WO-A-98/38994

## Beschreibung

Die Erfindung betrifft die Verwendung von 2-Methyl-thiazolidin-2,4-dicarbonsäure und/oder ihrer physiologisch verträglichen Salze zur Herstellung eines Medikaments zur Behandlung von Infektionen, die durch Retroviren hervorgerufen werden, insbesondere zur symptomatischen Behandlung.

Die Infektionen mit dem HI-Virus, der zu den Retro-Viren gehört, ist bis heute nicht heilbar. Die derzeitigen Therapien zielen darauf ab, die Zunahme der HI-Viren zu verhindem und die Symptome abzuschwächen. Aufgrund dieser Bemühungen ist es mittlerweile gelungen, das asymptomatische Stadium bei einer HIV-Infektion und damit auch die Lebenserwartung der Patienten wesentlich zu verlängern.

Die zur Therapie verwendeten Wirkstoffe weisen jedoch zum Teil beachtliche schädliche Nebenwirkungen auf. Zwar steigt die Lebenserwartung der Patienten, aber seine Lebensqualität nimmt durch die genannten Nebenwirkungen dramatisch ab.

Es ist daher Aufgabe der vorliegenden Erfindung, eine physiologisch gut verträgliche Substanz zur Herstellung eines Medikaments zur Behandlung von Infektionen mit Retroviren und insbesondere mit HI-Viren zur Verfügung zu stellen, die zudem weniger Nebenwirkungen aufweisen soll als die bisher verfügbaren Substanzen des Standes der Technik.

Diese Aufgabe wird durch die Verwendung von 2-Methyl-thiazolidin-2,4-dicarbonsäure (2-MTDC) und/oder ihrer physiologisch verträglichen Salze zur Herstellung eines Medikaments zur Behandlung von Infektionen mit Retro-Viren und insbesondere mit HI-Viren gelöst. und als adjuvante Therapie bei der Behandlung von HIV-Infektionen.

Die Synthese von 2-Methyl-thiazolidin-2,4-dicarbonsäure (2-MTDC), ihre Verwendung als Hepatoprotektivum sowie die Herstellung von 2-Methyl-thiazolidin-3,4-dicarbonsäure enthaltenden Arzneimitteln in Form von Dragees oder Salben ist aus DE-OS 21 16 629 bekannt. 2-MTDC ist für einige Anwendungen als Arzneimittel vorgesehen worden. So offenbart EP 989 16 811 die Verwendung von 2-Methylthiazolidin-2,4-dicarbonsäure (2-MTDC) als Mukolytikum und EP 989 16 809 beschreibt ein Kombinationspräparat aus 2-Methyl-thiazolidin-2,4-dicarbonsäure (2-MTDC) und Paracetamol.

Ober die therapeutischen Wirkungen bei mit Retroviren infizierten Personen war bisher nichts bekannt.

Überraschenderweise konnte gezeigt werden, daß durch die erfindungsgemäße Verwendung von 2-Methyl-thiazolidin-2,4-dicarbonsäure (2-MTDC) und/oder ihrer physiologisch verträglichen Salze eine Verminderung der Replikationsrate der Viren, eine Stärkung des Immunsystems des Betroffenen und gegebenenfalls eine Verhinderung bzw. eine Verlangsamung des Muskelschwunds in dem symptomatischen Stadium erzielt wird.

Diese Ergebnisse konnten durch Untersuchungen an Mäusen bestätigt werden. Der genaue Wirkmechanismus von 2-Methyl-thiazolidin-2,4-dicarbonsäure (2-MTDC) konnte bisher nicht eindeutig aufgeklärt werden. Es wird jedoch vermutet, daß 2-Methyl-thiazolidin-2,4-dicarbonsäure (2-MTDC) als "pro-drug" zur Freisetzng von L-Cystein dient, welches weiter zu Glutathion (γ-Glutamylcysteinylglycin, GSH) umgesetzt wird, dem die Funktion eines Radikalfängers zugeschrieben wird.

GSH ist die wichtigste natürliche Substanz, um die für die Körperzellen giftigen Sauerstoffradikale, die bei zahlreichen oxidativen Enzymreaktionen gebildet werden, abzufangen und zu beseitigen. GSH kommt in jeder Körperzelle in hohen Konzentrationen vor. Andernfalls würden die entstandenen Sauerstoffradikale Bestandteile der Zellmembran zerstören, aber auch andere intrazelluläre Vorgänge einschließlich Reparaturmechanismen im Bereich der Gene blockieren. Eine ungehinderte Wirkung der Sauerstoffradikale führt zum Zelluntergang.

Der für die Entgiftungsfunktion von GSH wichtigste Bestandteil ist die Aminosäure L-Cystein, die selbst schon in der Lage ist, Sauerstoffradikale zu inaktivieren. Bei einer HIV-Infektion wurde eine Verminderung der Blutspiegel von L-Cystein und Cystin, einem Abkömmling von L-Cystein, der in L-Cystein rückverwandelt werden kann, gefunden (Dröge W., Eck H.-P., Näher H., , Pekar U., Daniel V., Biol Chem Hoppe-Seyler 369: 143-148, 1988; Eck H.-P., Gmünder H., Hartmann M., Petzoldt D., Daniel V., Dröge W., Biol Chem Hoppe-Seyler 370, 101-108, 1989; Hortin G. L., Lundt M., Powderly W. G., Clin Chem 40: 785-789, 1994). Der deutlichste Abfall der L-Cystein-Spiegel findet im asymptomatischen Stadium einer HIV-Infektion statt, wenn die Zahl der CD4*T-Zellen stark abnimmt, dem sogenannten präkachektischen Stadium der HIV-Infektion. In diesem Stadium beginnt außerdem bereits ein meßbarer Verlust an Körperzellen.

Der Mangel an L-Cystein und Glutathion bei HIV-infizierten Patienten trägt wahrscheinlich dazu bei, die ohnehin beeinträchtige Immunabwehr dieser Patienten weiter zu schwächen. Die bereits geschilderte Koinzidenz zwischen der Abnahme der L-Cysteinkonzentration und der dramatischen Abnahme der CD4*T-Lymphozyten legt nahe, daß ein L-Cysteinmangel das Immunsystem schwächt.

In einer Reihe von Versuchsanordnungen konnte gezeigt werden, daß die intrazelluläre Konzentration von GSH entscheidend die Größe der immunologischen Funktionsfähigkeit bestimmt (Dröge W., Gross A., Hack V., Künscherf R., Schykowski M., Bockstette M., Galter D., Advances in Pharmacology 38: 581-600, 1995). Da L-Cystein die entscheidende Vorstufe für GSH in Lymphozyten ist, war zu erwarten, daß erniedrigte L-Cystein und Cystin-Spiegel bei HIV-Patienten mit einer Abnahme der GSH-Spiegel in Monozyten, Blutplasma und der Flüssigkeitsschicht in den Lungenbläschen einhergeht, was die Beobachtungen bestätigt haben.

Um das Defizit an L-Cystein bei HIV-infizierten Patienten auszugleichen, wurden diese bis zu vier Jahre lang mit dem zum Ausgleich von L-Cysteindefiziten eingeführten Medikament, nämlich N-Acetylcystein, behandelt. Diese Behandlung führte zu übernormal hohen L-Cysteinkonzentrationen im Blutplasma. Die Zahl der T-Lymphozyten blieb stabil. Allerdings gaben die Autoren das Risiko erhöhter Spiegel von L-Cystein und Cystin zu bedenken und stellten fest, daß die Dosis ständig an die Blutspiegel angepaßt werden müsse. Die Steuerbarkeit der N-Acetylcysteinmedikation stellte sich in diesen Langzeitstudien als Problem heraus.

Im Stadium der Kachexie (Auszehrung mit Muskelschwund) scheint ein gestörter L-Cysteinmetabolismus in der Skelettmuskulatur vorzuliegen, wodurch es zum Zelluntergang von Muskelzellen kommt (Dröge w., Gross A., Hack V., Künscherf R., Schykowski M., Bockstette M., Mihm S., Galter D., Advances in Pharmcology 38: 581-600, 1995).

Ein günstiger Effekt von GSH bzw. N-Acetylcystein wurde auch hinsichtlich der Virusreplikation gefunden. Dies wird damit erklärt, daß das Virus für seine Vermehrung einen natürlichen Faktor benötigt(NFκB), der durch Antioxidantien gehemmt wird, da er für seine Funktion reaktive Sauerstoffradikale benötigt.

Ein Grund für die Abnahme von L-Cystein in Immunzellen ist der hohe Bedarf an L-Cystein, um dieses in die viralen Proteine einzubauen (besonders Glykoprotein B).

Nach GSH-Behandlung ist die Zahl der freigesetzten Viren deutlich vermindert (Garaci E., Palamara A. T., Cirolo M. R., D'Agostini C., del-Latil M. S., Aquaro S., Lafavia E., Rotiio G., J Leukoc Biol 62: 54-59, 1997).

Diese Beobachtungen ließen erwarten, daß die rechtzeitige Substitution des L-Cystein- und Glutathiondefizits ein vielversprechender therapeutischer Ansatz bei HIV-infizierten Patienten ist.

Um diesen therapeutischen Ansatz zu realisieren, läge es nahe, die natürlichen Substanzen zu applizieren. Glutathion kommt nicht in Frage, weil es zum einen im Magen zerstört würde und nicht in die Zellen hineintransportiert werden kann. Die Zellen verfügen über keinen entsprechenden Transportmechanismus. L-Cystein ist giftig, wie dies in Zellkulturen und am Beispiel von neugeborenen Mäusen und Ratten mehrfach gezeigt worden ist. Die Substanzen müssen also durch Vorstufen substituiert werden, die im Körper in L-Cystein umgewandelt werden, das dann für die Synthese von Glutathion zur Verfügung steht. Die bekannteste Vorstufe ist N-Acetylcystein. Im allgemeinen wird davon ausgegangen, daß es sich bei N-Acetylcystein um ein Medikament geringer Toxizität handelt. Allerdings gibt es einige, wenig beachtete Berichte, daß das Toxizitätsrisiko von N-Acetylcystein unterschätzt wird(Estrela J. M., Saez G. T., Such L., Vina J., Biochem Pharmacol 32: 3483-3485, 1983 und Vina J., Romero F. J., Saez G. T., Pallarde F. V., Experimentia 39: 164-165, 1983). Außerdem wurde bereits erwähnt, daß die Langzeitbehandlung von HIV-infizierten Patienten mit N-Acetylcystein zu erheblichen Konzentrationsschwankungen im Blut führt, was dieses Medikament als ungeeignet für die Behandlung dieser Patienten wegen des relativ hohen Toxizitätsrisikos erscheinen läßt.

Wegen des Risikos toxischer Reaktionen wurde immer wieder nach Alternativen zu N-Acetylcystein gesucht. Ein weiteres Vorstufen-Medikament könnten Thiazolidine darstellen. Die Kondenstaion von Carbonyl-haltigen Stoffen mit L-Cystein zu Thiazolidinen wurde bereits beschrieben (Susilo, R., Rommelspacher H., Hoefle G., Journal of Neurochem 52: 1793-1800, 1989). Wichtig ist dabei, daß die Thiazolidine ein Reservoir für L-Cystein bilden, aus denen bei Bedarf die Aminosäure freigesetzt wird. Ein strukturell einfaches Thiazolidin ist das Kondensationsprodukt zwischen Formaldehyd und L-Cystein. Metaboliten dieser Substanz stellten sich allerdings als neurotoxisch heraus. Das Kondensationsprodukt zwischen Acetaldehyd und L-Cystein ist als Vorläufermedikament ebenfalls ungeeignet, da unter physiologischen Bedingungen leicht der relativ toxische Acetaldehyd gebildet wird (Wlodek L., Rommelspacher H., Susilo R., Radomski J., Hoefle G., Biochem Pharmacol 46: 917-928, 1993).

Es stellt daher ein überraschendes Ergebnis dar, daß durch die Verwendung von 2-Methyl-thiazolidin-2,4-dicarbonsäure (2-MTDC) die der Erfindung zugrunde liegende Aufgabe gelöst werden konnte.

Zudem wurde überraschenderweise gefunden, daß 2-Methylthiazolidin-2,4-dicarbonsäure (2-MTDC) eine Reduktion der Bildung der freien Radikale und eine Erhöhung des Sulfhydrylgruppen-Konzentration im Organismus hervorruft. Dies führt zu einer cytoprotektiven Wirkung dieser Verbindung.

Die Substanz ist den bisher bekannten Verbindungen, wie N-Acetylcystein deutlich überlegen. Die von N-Acetylcystein bekannten toxischen Nebenwirkungen können durch die Verwendung von 2-Methyl-thiazolidin-2,4-dicarbonsäure (2-MTDC) deutlich verringert werden.

Bei der Freisetzung von L-Cystein aus 2-Methylthiazolidin-2,4-dicarbonsäure (2-MTDC) entsteht als Nebenprodukt Pyruvat, eine physiologische Substanz, die völlig unschädlich ist. 2-Methyl-thiazolidin-2,4-dicarbonsäure (2-MTDC) zeichnet sich deshalb im Gegensatz zu N-Acetylcystein durch eine sehr gute Verträglichkeit aus.

Besonders vorteilhaft ist hierbei, daß es sogar Hinweise auf eine protektive Wirkung von Pyruvat gibt (Rastellini C., Cicalese L., Zeevi A., Matten C., Stauko R. T., Starzl T. E., Rao A. S., Transplantation Proceedings 27: 3383-3384, 1995). Pyruvat entsteht physiologischerweise aus Glucose und wird im Zitronensäurezyklus zur Energiegewinnung der Zelle benötigt.

Es wurde festgestellt, daß die Behandlung mit 2-Methylthiazolidin-2,4-dicarbonsäure (2-MTDC) nicht nur die Verhinderung der Zerstörung des zellulären Abwehrsystems am Beispiel von Leberzellen bewirkt (Wlodek L., Rommelspacher H., Alcohol and Alcoholism 29: 649-657, 1994), sondern auch ein lang anhaltender Effekt der Wirkung von 2-MTDC erzielt wird.

Die Freisetzung von L-Cystein und die Bildung von GSH war noch nach 12 Stunden im Lebergewebe von Mäusen nachweisbar. 2-MTDC 1,2 mmol/kg, intraperitoneal appliziert, führte nach 12 Stunden zu einer Erhöhung des GSH-Spiegels auf 112,0 % (P<0,01). 2-MTDC, 2,4 mmol/kg, intraperitoneal appliziert,. führte zu einer Erhöhung des GSH-Spiegels in der Leber auf 154,5 % der Kontrollwerte, P<0,001; Wlodek L., Rommelspacher H., Fundamentals in Clinical Pharmacology 11: 454-459, 1997, Tabelle 1).

Diese Beobachtungen sprechen für eine enzymatisch regulierte Freisetzung von L-Cystein aus 2-MTDC. Dies führt zu einer dem Bedarf des Körpers angepaßten dosisabhängigen Freisetzung von L-Cystein. Dadurch werden sehr hohe Konzentrationen von L-Cystein, wie nach der Dauertherapie mit N-Acetylcystein beobachtet, vermieden. Dies trägt ebenfalls zur besseren Verträglichkeit von 2-MTDC bei.

Aufgrund dieser Beobachtungen ist zu erwarten, daß die orale und systemische Gabe von 2-MTDC die immunologische Funktionsfähigkeit des HIV-Patienten, die Verhinderung bzw. Reduktion des Zelluntergangs im Stadium der Kachexie sowie die Hemmung der Virusreplikation günstig beeinflussen.

Eine rechtzeitige Therapie mit 2-MTDC ist daher ein vielversprechender Ansatz zur Behandlung von HIV-infizierten Patienten.

## Patentansprüche

1. Verwendung von 2-Methyl-thiazolidin-2,4-dicarbonsäure und/oder ihrer physiologisch verträglichen Salze zur Herstellung eines Medikaments zur Behandlung von Infektionen mit Retro-Viren.

2. Verwendung von 2-Methyl-thiazolidin-2,4-dicarbonsäure und/oder ihrer physiologisch verträglichen Salze nach Anspruch 1 zur Herstellung eines Medikaments zur Behandlung von HIV-Infektionen.

3. Verwendung von 2-Methyl-thiazolidin-2,4-dicarbonsäure und/oder ihrer physiologisch verträglichen Salze nach Anspruch 1 zur Herstellung eines Medikaments zur symptomatischen Behandlung von Infektionen mit Retro-Viren.

4. Verwendung von 2-Methyl-thiazolidin-2,4-dicarbonsäure und/oder ihrer physiologisch verträglichen Salze nach Anspruch 2 zur Herstellung eines Medikaments zur symptomatischen Behandlung von HIV-Infektionen.

5. Verwendung von 2-Methyl-thiazolidin-2,4-dicarbonsäure und/oder ihrer physiologisch verträglichen Salze nach Anspruch 2 zur Herstellung eines Medikaments als adjuvante Therapie bei der Behandlung von HIV-Infektionen.

## Claims

1. Use of 2-methyl-thiazolidine-2,4-dicarboxylic acid and/or its physiologically acceptable salts for manufacturing a medicament for the treatment of retrovirus infections.

2. Use of 2-methyl-thiazolidine-2,4-dicarboxylic acid and/or its physiologically acceptable salts according to claim 1 for manufacturing a medicament for the treatment of HIV infections.

3. Use of 2-methyl-thiazolidine-2,4-dicarboxylic acid and/or its physiologically acceptable salts according to claim 1 for manufacturing a medicament for the symptomatic treatment of retrovirus infections.

4. Use of 2-methyl-thiazolidine-2,4-dicarboxylic acid and/or its physiologically acceptable salts according to claim 2 for manufacturing a medicament for the symptomatic treatment of HIV infections.

5. Use of 2-methyl-thiazolidine-2,4-dicarboxylic acid and/or its physiologically acceptable salts according to claim 2 for manufacturing a medicament as adjuvant therapy for the treatment of HIV infections.

## Revendications

1. Utilisation d'acide 2-méthyl-thiazolidine-2,4-dicarboxylique et/ou de ses sels physiologiquement acceptables pour la préparation d'un médicament pour le traitement des infections par des rétro-virus.

2. Utilisation d'acide 2-méthyl-thiazolidine-2,4-dicarboxylique et/ou de ses sels physiologiquement acceptables selon la revendication 1 pour la préparation d'un médicament pour le traitement des infections par le VIH.

3. Utilisation d'acide 2-méthyl-thiazolidine-2,4-dicarboxylique et/ou de ses sels physiologiquement acceptables selon la revendication 1 pour la préparation d'un médicament pour le traitement symptomatique des infections par des rétro-virus.

4. Utilisation d'acide 2-méthyl-thiazolidine-2,4-dicarboxylique et/ou de ses sels physiologiquement acceptables selon la revendication 2 pour la préparation d'un médicament pour le traitement symptomatique des infections par le VIH.

5. Utilisation d'acide 2-méthyl-thiazolidine-2,4-dicarboxylique et/ou de ses sels physiologiquement acceptables selon la revendication 2 pour la préparation d'un médicament pour comme thérapie adjuvante lors du traitement des infections par le VIH.
